# EUROPÄISCHE PATENTANMELDUNG

(11) **EP 0 761 677 A2**
(43) Veröffentlichungstag der Anmeldung: **12.03.1997**
(21) Anmeldenummer: 96107034.9
(22) Anmeldetag: 04.05.1996
(51) Int. Cl.: C07H 15/08, C07H 17/04, C11D 1/66, B01F 17/00

(54) **Sauer spaltbare Tenside auf Basis von Alkylglykosiden**

(30) Priorität: 08.07.1995 DE 19524973
(71) Anmelder: HÜLS AKTIENGESELLSCHAFT, 45764 Marl (DE)
(72) Erfinder: Koch, Herbert, Dr., 46282 Dorsten (DE); Ruback, Wulf, Dr., 48249 Dülmen (DE); Schröder, Wolfgang, Dr., 46282 Dorsten (DE)

(57) **Zusammenfassung**

Die Erfindung betrifft Umsetzungsprodukte von Alkylglykosiden mit Aldehyden und/oder Diacetalen von kurzkettigen Alkoholen und Aldehyden, wobei die gewonnenen Acetale alkoxyliert werden. Weiterhin betrifft die Erfindung ein Verfahren zur Herstellung der obengenannten Verbindungen sowie die Verwendung dieser Verbindungen zur Herstellung oberflächenaktiver Mittel, insbesondere für industrielle Wasch- und Reinigungsmittel.

## Beschreibung

Die Erfindung betrifft Umsetzungsprodukte von Alkylglykosiden mit Aldehyden und/oder Diacetalen von kurzkettigen Alkoholen und Aldehyden, wobei die gewonnenen Acetale alkoxyliert werden. Weiterhin betrifft die Erfindung ein Verfahren zur Herstellung der obengenannten Verbindungen sowie die Verwendung dieser Verbindungen zur Herstellung oberflächenaktiver Mittel, insbesondere für industrielle Wasch- und Reinigungsmittel.

Die erfindungsgemäßen Alkylglykosidtenside nach Formel I sind alkalistabil, schaumarm, spaltbar in biologisch abbaubare Fragmente und zeigen insgesamt ein gutes Netzvermögen harter Oberflächen.

Mit dem Verzicht auf Lösemittel, insbesondere chlorierte Kohlenwasserstoffe, deren Einsatz bei der Tauchreinigung zur Entfernung von Fetten nach der 2. BImSchV drastisch eingeschränkt wurde, geht man heute verstärkt dazu über, in vielen technischen Anwendungen wäßrige, tensidhaltige Systeme einzusetzen. Deren Standzeit gegen mikrobiellen Angriff ist jedoch ein vordringliches Problem. Zur Zeit wird dieses entweder durch (Nach)Dosierung erheblicher Mengen Bakterizide oder durch Einsatz schwer- bis nicht abbaubarer Einsatzstoffe - in erster Linie Tenside - gelöst.

Ein Lösungsansatz für dieses Problem ist der Einsatz spaltbarer Tenside, die in ihrer Anwendungsform zuerst einmal biologisch hart, d.h. nicht abbaubar sind und nach ihrem Einsatz z.B. in Metallbädern oder allgemein bei der Reinigung von harten Oberflächen durch einfache Operationen in biologisch abbaubare Fragmente überführt werden. Ein weiteres Ziel ist es, durch die Spaltung der Tenside eine wäßrige und eine organische Phase zu erhalten, derart, daß die organische Phase mit dem Fettschmutz abgetrennt werden kann, wodurch die Fracht an organischen Abfällen im Abwasser reduziert werden kann. Unter gewissen Umständen kann es sogar von ökonomischem Interesse sein, die abgetrennte organische Fracht aufzuarbeiten, um dadurch Hertstoffe zurückzugewinnen ("rent a chemical").

Das chemische Konzept sieht hierfür in erster Linie verzweigte Tenside vor, die eine pH-sensitive Funktion als sogenannte Sollbruchstelle enthalten. Durch pH-Änderung kann das Tensid in biologisch abbaubare Fragmente gespalten werden und gleichzeitig eine Phasenseparierung in eine organische Phase mit hydrophoben Schmutzpartikeln und lipophilen Tensidbestandteilen und eine wäßrige Phase erzielt werden.

Das Prinzip der pH-sensitiven Tenside ist schon seit mehreren Jahren Gegenstand verschiedener Veröffentlichungen und Patente.

In Industrial Launderer (Juli 1990, S. 41 f) sowie in dem technischen Merkblatt Triton RW-Surfactant (Juli 1982) wird beschrieben, stabile Emulsionen von Ölen und Abwasser, die pH-sensitive Tenside enthalten, durch Zugabe von Säuren zu brechen und die Ölphase abzutrennen. Durch diesen Prozeß erhält man weitgehend ölfreie Abwässer. Bei den hier zum Einsatz gelangenden Tensiden handelt es sich um Ethoxylierungsprodukte von primären Aminen. Produkte dieser Art sind zwar für ihre gute Reinigungsleistung bekannt, jedoch biologisch nicht abbaubar.

In DE 42 27 894 wird ein Verfahren zur Reduktion organischer Bestandteile in Abwässern aus gewerblichen Wäschereien beschrieben, bei dem diverse verzweigte Alkanolamin/amidethoxylate eingesetzt werden. Die dort beschriebenen pH-sensitiven Tenside sollen sich durch eine hohe Reinigungsleistung, eine gute Separierung aus den Emulsionen bei pH-Änderung sowie durch ihre gute biologische Abbaubarkeit auszeichnen. Ein Nachteil ist der Einsatz von Amin-Vorprodukten zur Synthese dieser Verbindungen und die damit verbundene Nitrosaminproblematik.

Jaeger et. al. beschreiben in JACS 111, S. 3001 - 3006 verzweigte Acetaltenside, die durch Umsetzung von langkettigen Fettketonen mit Glycerin hergestellt werden.

Sokolowski, Piasecki et. al. beschreiben in Tenside, Surf., Det. 30 (1993) S. 417 spaltbare Tenside, die als pH-sensitive Funktion eine Acetalgruppe aufweisen. Die Acetalgruppe zeichnet sich im basischen Milieu durch eine hohe Stabilität aus, während im sauren pH-Bereich eine Spaltung auftritt. Nachteil der hier beschriebenen Verbindungen sind die teuren Einsatzstoffe.

Der Erfindung liegt daher die Aufgabe zugrunde, spaltbare Tenside zur Verfügung zu stellen, die frei von den geschilderten Nachteilen sind.

Als Edukte sollen dabei möglichst gut verfügbare, billige Einsatzstoffe, nach Möglichkeit auf Basis nachwachsender Rohstoffe, gewählt werden.

Die spaltbaren Tenside sollen weiterhin im Hinblick auf ihre Anwendung in technischen Reinigungsprozessen folgendes Eigenschaftsprofil aufweisen:
1. Schaumarmut
2. Alkalistabilität
3. "Biologische Härte" in ihrer Anwendungsform
4. Im sauren Bereich Spaltbarkeit in biologisch abbaubare Teilstrukturen
5. Hohe Reinigungsleistung besonders harter Oberflächen

Die Aufgabe wird erfindungsgemäß gelöst durch sauer spaltbare Tenside auf Alkylglykosidbasis. Die Erfindung betrifft daher sauer spaltbare Tenside auf Alkylglykosidbasis nach der allgemeinen Formel I, 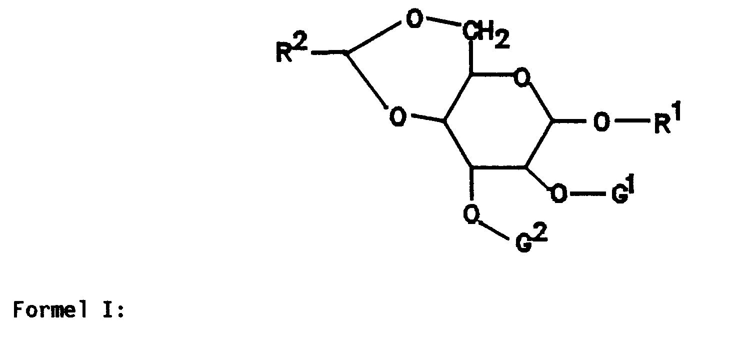 in der R¹ für unverzweigte oder verzweigte Alkyl- und/oder Alkylengruppen mit 1 bis 20 Kohlenstoff-Atomen,
R² für Alkylgruppen mit 6 bis 20 Kohlenstoff-Atomen,
G¹ für (EO)ᵥ(PO)_{w}H und G² für (EO)ₓ(PO)_{y}H mit EO = Ethylenoxy-, PO = Propylenoxygruppe, stehen und
v + w = 0-30, x + y = 0-30; v + w + x + y >= 1 ist.

Die Erfindung betrifft weiterhin ein Verfahren zur Herstellung der sauer spaltbaren Tenside, ein Verfahren zur Reinigung in technischen Prozessen unter Einsatz dieser sauer spaltbaren Tenside und ihre Verwendung.

Als unverzweigte oder verzweigte Alkyl- und Alkenylgruppen R¹ seien beispielsweise die Methyl-, Ethyl-, n-Propyl-, Isopropyl-, n-Butyl-, iso-Butyl-, tert.Butyl-, n-Pentyl-, Neopentyl-, n-Hexyl-, n-Heptyl-, n-Octyl-, 2-Ethylhexyl-, n-Nonyl-, Isononyl-, n-Decyl-, Isodecyl-, n-Undecyl-, n-Dodecyl-, n-Tridecyl-, Isotridecyl-, n-Tetradecyl-, n-Pentadecyl-, n-Hexadecyl-, n-Heptadecyl-, n-Octadecyl-, n-Eicosyl-, Oleyl-, Linolyl- und Linolenyl-Gruppen genannt. Bei den hier beschriebenen Alkyl- und Alkenylgruppen kann es sich natürlich auch um die entsprechenden Mischungen handeln. Vorzugsweise handelt es sich bei den Resten R¹ um gesättigte und wenig verzweigte Alkylgruppen mit 1-14 Kohlenstoff-Atomen.

Besonders bevorzugt sind Methyl-, Butyl- sowie unverzweigte C₈-C₁₄ Alkylgruppen, die in Form ihrer Glykoside kommerziell erhältlich sind.

Zweckmäßigerweise stellt man die Verbindungen nach Formel I dadurch her, daß man Alkylglykoside mit Aldehyden bzw. Diacetalen bei Temperaturen von 50 - 200 °C unter Vakuum in Gegenwart katalytischer Mengen Säure umsetzt. Dabei bilden sich cyclische Acetale zwischen dem Aldehyd-Kohlenstoff-Atom und den Glykosid-OH-Gruppen in 4 und 6 Position des Glykosids aus (siehe Formel II). Eine andere Moglichkeit zur Synthese der erfindungsgemäßen Tensidverbindungen nach Formel I besteht in der Umacetalisierung von Fettaldehyddiacetalen aus Fettaldehyden und kurzkettigen Alkoholen mit den entsprechenden Alkylglykosiden. Diese Art der Umsetzung kann unter schonenderen Bedingungen durchgeführt werden als die direkte Acetalisierung.

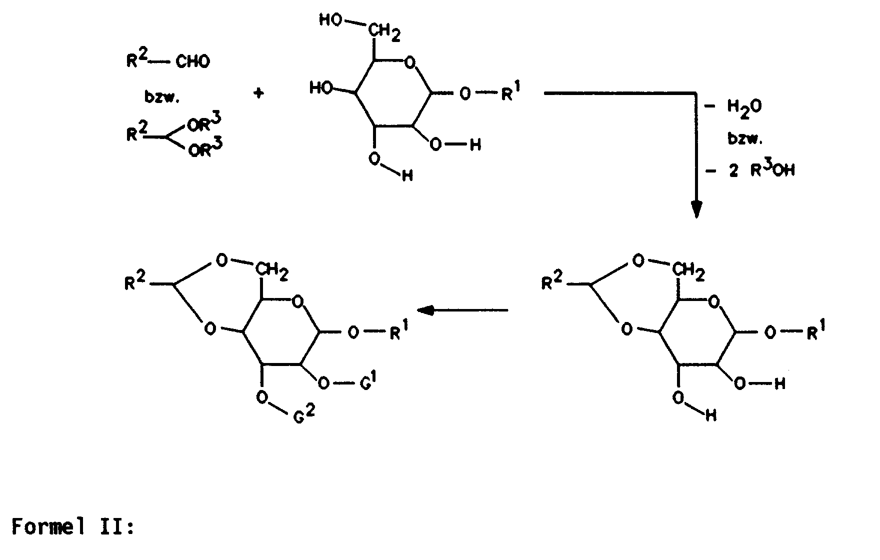

Die hierbei eingesetzten Aldehyde sind Vertreter mit gesättigter C₆-C₂₀-Alkylkette. Als Beispiele seien genannt: n-Hexanal, n-Heptanal, n-Octanal, 2-Ethylhexanal, n-Nonanal, n-Decanal, n-Undecanal, n-Dodecanal. Weitere Vertreter sind sämtliche Homologe mit verzweigter C₆-C₂₀ Kohlenstoffkette. Diese sind z.B. durch Hydroformylierung von Olefinen mit innenständiger Doppelbindung zugänglich. Da die hierbei eingesetzten Olefine in der Regel eine statistische Homologen- und Isomerenverteilung aufweisen, ist es zweckmäßig, bei den Resten R² von einer durchschnittlichen Anzahl der Kohlenstoff-Atome zu sprechen.

Bei dem Rest R³ handelt es sich um kurze, unverzweigte oder verzweigte Alkylgruppen wie z. B. die Methyl-, Ethyl-, Propyl-, Butylgruppe.

Neben einer Direktacetalisierung kann, wie oben beschrieben, auch eine Umacetalisierung durchgeführt werden. Zu diesem Zweck werden die Aldehyde zuerst mit Alkoholen in die entsprechenden Diacetale überführt. Gewöhnlich setzt man hier kürzere Alkohole ein, da die Reaktion meist in einem Überschuß an Alkohol durchgeführt wird, der zum Schluß destillativ entfernt wird. Eine weitere Möglichkeit zur Herstellung von Dimethyl- bzw. Diethylacetalen ist die Umsetzung der beschriebenen Aldehyde mit Orthoestern (Houben-Weyl, Bd. 7, 4. Auflage, S. 417 ff.). Da diese Acetalisierungsverfahren Stand der Technik sind, soll an dieser Stelle nicht näher darauf eingegangen werden.

Die Acetalisierung der Aldehyde bzw. die Umacetalisierung der Aldehyddiacetale mit den beschriebenen Alkylglykosiden kann lösungsmittelfrei durchgeführt werden. Ein zusätzliches inertes Lösungs- oder Verdünnungsmittel ist normalerweise nicht notwendig, kann aber im Bedarfsfall, beispielsweise bei Viskositätsproblemen, zugesetzt werden.

Als Katalysatoren eignen sich Mineralsäuren wie z.B. HCl, H₂SO₄, H₃PO₄ oder HClO₄; organische Carbon- und Sulfonsäuren, wie z.B. Methansulfonsäure, p-Toluolsulfonsäure, Oxalsäure, Ameisensäure, Essigsäure, Propionsäure, oder Lewissäuren wie z.B. BF₃, AlCl₃, ZnCl₂ oder TiCl₄. Bei der schonenderen Umacetalisierung können auch saure Tonminerale, wie z. B. K10 eingesetzt werden. Besonders vorteilhaft läßt sich p-Toluolsulfonsäure als Katalysator verwenden. Der saure Katalysator wird in den hierbei üblichen Mengen zugesetzt, also normalerweise in einer Menge von etwa 0,1 - 5 mol%, bezogen auf die Aldehydkomponente. Eine Neutralisation des sauren Katalysators nach erfolgter Umsetzung kann mit anorganischen Basen, z. B. NaOH, KOH, K₂CO₃, Na₂CO₃, oder organischen Basen, z. B. Trimethylamin, Triethylamin, Dimethylcyclohexylamin oder Pyridin durchgeführt werden. Grundsätzlich ist eine separate Neutralisation nicht notwendig, da im Folgeschritt die Alkoxylierung des Grundkörpers unter basischer Katalyse durchgeführt wird.

Der Verlauf der Acetalisierung/Umacetalisierung kann anhand der gewonnenen Destillatmengen an Wasser/Alkohol verfolgt und quantifiziert werden.

Die gewonnenen Produkte können als Nebenbestandteile gewisse Mengen an höheren Oligomeren, sog. Oligoglykoside enthalten.

Im Folgeschritt werden die Verbindungen alkoxyliert. Die Umsetzung erfolgt mit Ethylen- und/oder Propylenoxid (EO/PO) in bekannter Art und Weise. Dabei können statistische Gemische aus EO/PO oder eine aus bis zu drei einheitlichen Blöcken dieser Alkylengruppen aufgebauten Gruppe entstehen.

Die Alkoxylierungsgrade liegen zwischen > 0 und 30, vorzugsweise 5 und 20. Die Werte für v + w bzw. x + y stellen üblicherweise Durchschnittswerte dar.

Die pH-sensitiven Tenside nach Formel I eignen sich generell als oberflächenaktive Substanzen für industrielle Anwendungszwecke und weisen eine Vielzahl von technischen Anwendungsmöglichkeiten auf. Besonders hervorzuheben ist ihr Einsatz in Reinigungsbädern der Metallindustrie zur Entfettung von Metallteilen sowie bei der industriellen Reinigung von Glasflaschen, insbesondere also bei maschinell ablaufenden Reinigungsprozessen.

Für diese Zwecke werden insbesondere Tenside benötigt, die den hochalkalischen Bedingungen der Reinigungsbäder standhalten, die schwach schäumend sind und sich durch eine gute Benetzung harter Oberflächen auszeichnen.

Die Konzentration der sauer spaltbaren Tenside beträgt 0,1 bis 70 Gew.-%, bezogen auf die Zubereitung.

Durch Einstellung der Reinigungsbäder auf einen sauren pH-Bereich von 0,5 - 6 werden die hier beanspruchten Tenside gespalten, sie verlieren ihren tensidischen Charakter, wodurch die Schmutzemulsionen brechen und zwei Phasen bilden.

### Herstellungsbeispiele

### Beispiel 1

490 g (2,5 mol) Methyl-α-D-glucopyranosid und 575 g (2,5 mol) Laurinaldehyddimethylacetal wurden in einem 2 l Dreihalskolben unter Schutzgas vorgelegt. Nach Zusatz von 1,0 g p-Toluolsulfonsäure wurde der Reaktionsansatz langsam auf Temperaturen von 60 - 90 °C aufgeheizt und das gebildete

Methanol aufgefangen. Um einen gleichmäßigen Anfall an Methanol und somit einen gleichmäßigen Reaktionsverlauf zu gewährleisten, wurde ein Vakuum angelegt und dieses sukzessive je nach Destillatanfall bis auf 20 mbar gesenkt. Nach 3 - 6 Stunden war die theoretische Methanolmenge angefallen.

Nach Überführung in einen Autoklaven wurde das Reaktionsprodukt mit 0,1 - 0,2 Gew.% Natriumhydroxid versetzt und vorsichtig entwässert. Danach erfolgt eine Umsetzung mit Ethylenoxid (ca. 1100 g = ca. 10 mol EO/mol Einsatzstoff) bei Temperaturen von 90 - 140 °C. Nachdem die gewünschte Menge an Ethylenoxid aufgenommen worden war, ließ man den Reaktionsansatz abkühlen, neutralisierte mit Milchsäure und filtrierte zum Schluß heiß.

### Beispiel 2

293 g (0,31 mol) einer butanolischen Butylglycosidlösung (28%ig) und 57 g (0,31 mol) Laurinaldehyd wurden in einem 2 l Dreihalskolben unter Schutzgas vorgelegt. Nach Zusatz von 1,8 g p-Toluolsulfonsäure wurde der Reaktionsansatz langsam auf Temperaturen von 60 - 90 °C aufgeheizt und bei einem Vakuum von 250 - 20 mbar das gebildete Reaktionswasser azeotrop mit Butanol aufgefangen. Nachdem die theoretische Menge an Wasser abgespalten war, wurde das Restbutanol kondensiert und das Reaktionsprodukt in einen Autoklaven überführt. Nach Zusatz von 0,1 - 0,2 Gew.% Natriumhydroxid wurde vorsichtig entwässert. Danach erfolgte eine Umsetzung mit Ethylenoxid analog zu Beispiel 1 mit ca. 10 mol EO/mol Einsatzstoff.

### Anwendungstechnische Eigenschaften

Schäumvermögen und Schaumstabilität wurden nach DIN 53902 in Trinkwasser (TW) bei 20 und 60 °C von wäßrigen Mischungen mit 0,1 g Aktivgehalt/l bestimmt. Das Gefäß (zylindrische Form, Volumen 1000 ml) wurde jeweils mit einem Volumen von 200 ml aufgefüllt. Die Schaumhöhen wurden nach 60 Schlägen und anschließender Ruhezeit von 30 und 300 Sekunden abgelesen.

Aus den Ergebnissen (siehe Tabelle) geht hervor, daß nach dem Schaumtest die untersuchten Verbindungen durchweg als sehr schwache Schäumer einzustufen sind.

Das Netzvermögen wurde in Anlehnung an DIN-ISO 8022 bestimmt. Dazu wurden jeweils Konzentrationen von 1,0 g Aktivgehalt/l Trinkwasser bei 20 und 60 °C untersucht, wobei die angegebenen Werte jeweils Mittelwerte von 10 Messungen darstellen.

Zur Bestimmung der Randwinkel auf PP siehe "Seifen-Öle-Fette-Wachse -108. Jg. - Nr. 15/1982".

**Tabelle**

| Verbindung | Schaufflest Schaumhöhen (in ml) | | | | Netztest | | Randwinkelabnahme auf PP in VE-Wasser (in %) | | |
|---|---|---|---|---|---|---|---|---|---|
| | bei 20 °C | | bei 60 °C | | Untersinkzeit (sec.) in TW | | Konz. 0,1g/l | Konz. 1,0g/l | Konz. 10 g/l |
| | 30 sec. | 300 sec. | 30 sec. | 300 sec. | 20 °C | 60 °C | | | |
| **Bsp 1** | 60 | 50 | 20 | 10 | >300 | 85 | 20 | 46 | 48 |
| **Bsp 2** | 50 | 40 | 10 | 0 | 59 | 49 | 21 | 35 | 41 |

## Patentansprüche

1. Sauer spaltbare Tenside auf Alkylglycosidbasis nach der allgemeinen Formel I, 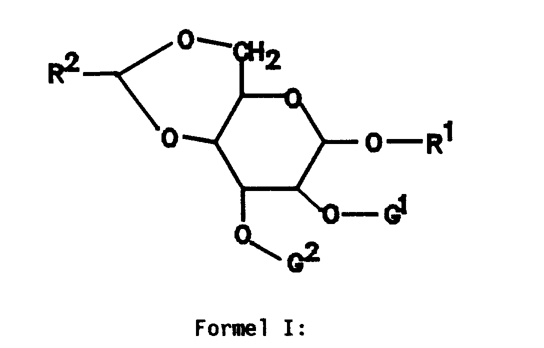 in der R¹ für unverzweigte oder verzweigte Alkyl- und/oder Alkylengruppen mit 1 bis 20 Kohlenstoff-Atomen,
R² für Alkylgruppen mit 6 bis 20 Kohlenstoff-Atomen,
G¹ für (EO)ᵥ(PO)_{w}H und G² für (EO)ₓ(PO)_{y}H mit EO = Ethylenoxy-, PO = Propylenoxygruppe, stehen und
v + w = 0-30, x + y = 0-30; v + w + x + y >= 1 ist.

2. Sauer spaltbare Tenside nach Anspruch 1,
dadurch gekennzeichnet,
daß R¹ Alkylgruppen mit 1-14 Kohlenstoff-Atomen bedeutet.

3. Sauer spaltbare Tenside nach einem der vorhergehenden Ansprüche,
dadurch gekennzeichnet,
daß G¹ und G² Ethylenoxid-Einheiten darstellen.

4. Sauer spaltbare Tenside nach einem der vorhergehenden Ansprüche,
dadurch gekennzeichnet,
daß v + x = 5 bis 20 und w = y = 0 ist.

5. Verfahren zur Herstellung spaltbarer Tenside nach einem der Ansprüche 1 bis 4,
dadurch gekennzeichnet,
daß man Alkylglycoside mit Aldehyden bzw. Diacetalen von Aldehyden und kurzkettigen Alkoholen bei Temperaturen von 50 - 200 °C in Gegenwart katalytischer Mengen an Säure acetalisiert/umacetalisiert und anschließend mit Ethylen- und/oder Propylenoxid unter Zusatz eines Katalysators alkoxyliert.

6. Verfahren zur Reinigung in technischen Prozessen,
dadurch gekennzeichnet,
daß man sauer spaltbare Tenside nach einem der Ansprüche 1 bis 4 einsetzt.

7. Verwendung von sauer spaltbaren Tensiden nach einem der Ansprüche 1 bis 4 für industrielle Anwendungszwecke.

8. Verwendung von sauer spaltbaren Tensiden nach einem der Ansprüche 1 bis 4 in Wasch- und Reinigungsmitteln.

9. Verwendung von sauer spaltbaren Tensiden nach einem der Ansprüche 1 bis 4 bei maschinell ablaufenden Reinigungsprozessen.

10. Verwendung von 0,1 bis 70 Gew.-%, bezogen auf die Gesamtmenge der Zubereitung, sauer spaltbaren Tensiden nach einem der Ansprüche 1 bis 4 neben üblichen Bestandteilen in Wasch- und Reinigungsmitteln.
